# EUROPEAN PATENT SPECIFICATION

(11) **EP 0 328 162 B1**
(45) Date of publication and mention of the grant of the patent: **10.02.1993**
(21) Application number: 89105436.3
(22) Date of filing: 10.04.1984
(51) Int. Cl.: A61M 5/14

(54) **Fault detection apparatus for parenteral infusion system and method of detecting a fault in a parenteral infusion system**
Überwachungsgerät für parentale Infusionen und Verfahren zur Überwachung eines eine Flüssigkeit paranteral zuführenden Systems
Appareil de détection de faute pour système de perfusion parentérale et procédé de détection de défauts au cours du fonctionnement d'un système

(30) Priority: 11.04.1983 US 483903
(43) Date of publication of application: 16.08.1989
(62) Divisional of application: 84103975.3
(73) Proprietor: IVAC CORPORATION, San Diego, CA 92121 (US)
(72) Inventor: Nelson, Peter E., Mountain View California (US)
(74) Representative: Howden, Christopher Andrew

(56) References cited:
- EP-A- 0 096 849
- FR-A- 2 091 101
- US-A- 3 690 318
- US-A- 4 392 847

## Description

This invention relates generally to systems for administering parenteral fluids to a patient, and, more particularly, to systems of this type having an infusior apparatus for infusing the fluid into the patient's vascular system.

Systems of this particular type have enjoyed widespread usage in hospitals for administering parenteral fluids at precise rates. The systems are useful for both venous and arterial infusions and typically include an infusion pump and an associated controlling device for pumping the parenteral fluid through a fluid tube and needle to the patient's vein or artery.

One drawback to conventional infusion pump systems of this type is that the needle can sometimes become dislodged from the patient's vein or artery. This will normally cause an increase in back pressure, but the pump will nevertheless continue to pump fluid at substantially the same fixed rate. The fluid therefore can infiltrate into the patient's body tissue and cause severe damage. Similarly, the needle can sometimes become dislodged from the patient entirely, yet the pump will continue to pump the fluid at the same fixed rate.

One known prior technique for detecting fluid infiltrations is to monitor the patient's skin temperature in the vicinity of the needle. Since the parenteral fluid is ordinarily cooler than the patient's body temperature, and since the fluid is not carried away as rapidly when an infiltration occurs, an infiltration will ordinarily create a temperature drop in the vicinity of the needle. Thus, whenever a drop in skin temperature is detected, it is deduced that an infiltration is occurring. This technique is not believed to have proven completely satisfactorily in all circumstances, such as, for example, when the parenteral fluid has a temperature substantially the same as that of the patient's blood.

Other known prior techniques for detecting an infiltration of a parenteral fluid into a patient s body tissue involve intervention by hospital personnel. In one such technique, an attendant visually inspects the region around the needle, to detect any swelling that might indicate an infiltration. In another technique, useful only when the fluid is being administered from a bottle under the force of gravity, the attendant periodically lowers the bottle to an elevation below the needle such that fluid flows outwardly from the patient. If when this is done the patient's blood does not appear in the fluid tube, it can be deduced that the needle is not in fluid communication with a vein or artery. Neither of these techniques has proven to be entirely satisfactory, one reason being that they both require the presence of trained hospital personnel and cannot be performed automatically.

Still another prior technique for detecting infiltrations and other fault conditions is used in a parenteral administration system that regulates flow rate using a pinch valve located in the fluid tube, between a drop chamber and the patient. In particular, the pinch valve is controllably adjusted in order to maintain the frequency of fluid drops into the drop chamber at a selected value. If the limits of the pinch valve are exceeded in attempting to maintain the selected drop frequency, it is deduced that a fault condition is present. Operator intervention is still required, however, in order to determine the particular type of fault condition, e.g. an infiltration, that is present.

EP-A-0096849, published 28th December 1983, which forms the basis for the preamble of Claims 1 and 2, discloses a method and apparatus for detecting occlusions in a parental administration system of a type having a peristaltic pump for pumping a fluid through a feeding tube to a patient. A pressure transducer measures the cyclicly varying pressure of the fluid in the feeding tube and produces a corresponding pressure signal, and a comparator compares the pressure signal to a selected one of several thresholds, depending on the current stage of the pumping cycle. An occlusion alarm is actuated whenever the selected threshold is exceeded. The device of EP-A-0096849 is still, however, limited in its applications.

DE-A-3018641 discloses a parenteral infusion system having a peristaltic pump for infusing fluid into a vein of a patient. A pressure operated switch is operated when the pressure of the fluid supplied to the patient exceeds a predetermined level. Similarly, a flow sensor senses the rate of flow of infusion fluid to the patient. The apparatus operates an alarm and stops the peristaltic pump when the pressure switch is not operated and the flow rate is zero; when the flow rate is zero and the pressure switch is operated or when the pressure switch is not operated but the flow rate is high. In the apparatus of DE-A-3018641, however, the pressure operated switch is simply a two-state device being in a first state when the pressure is below a predetermined threshold and in a second state when the pressure is above the predetermined threshold.

US-A-4277227 discloses apparatus which monitors the pressure of intravenous fluid supplied to a patient and acts to terminate such supply, by stopping the infusion pump, when a predetermined threshold is exceeded. Once again, the device is a simple two-state mechanism, being in one state when the pressure is above a pre-set threshold and another state when the pressure is below that threshold.

It should be appreciated from the foregoing that there still is a need for an effective method and apparatus for automatically detecting faults such as an open line in a parenteral administration system of the type having an infusion device. The present invention fulfils this need.

According to one aspect of the present invention, there is provided a parenteral administration system having an infusion device for infusing parenteral fluid in a pulsing fashion through a fluid tube into the vascular system of a patient and having a fault detection apparatus which includes a pressure transducer means for monitoring the pressure of the fluid in the fluid tube and producing a corresponding pressure signal and a processor for evaluating the pressure signal to detect the existence of a fault; characterised in that the processor includes filter means for high-pass filtering the pressure signal to produce an a.c. pressure signal comprising a positive signal excursion occurring with each delivery pulse of the infusion device followed by a negative signal excursion, and comparison means for comparing the magnitude of the negative signal excursion with a threshold comprising a predetermined proportion of the peak magnitude of the preceding positive signal excursion and alarm means for producing an alarm signal whenever the magnitude of the negative signal excursion exceeds said threshold.

According to another aspect of the present invention, there is provided a method of detecting a fault in a fluid infusion system, the infusion system having an infusion device for infusing parenteral fluid in a pulsing fashion through a fluid tube and further having pressure transducer means for monitoring the pressure of the fluid in the fluid tube and for producing a corresponding pressure signal characterised by the steps of:
high-pass filtering the pressure signal to produce an a.c. pressure signal comprising a positive signal excursion occurring with each delivery pulse of the infusion device followed by a negative signal excursion;
determining a threshold by taking a predetermined proportion of the peak magnitude of said positive signal excursion;
comparing the magnitude of the negative signal excursion with the threshold; and
producing an alarm signal whenever the magnitude of the negative signal excursion exceeds said threshold.

An embodiment of the present invention is described below by way of example with reference to the accompanying drawings in which:-
FIGURE 1 is a block diagram of a parenteral administration system having circuitry for detecting venous infiltrations, arterial infiltrations and open fluid lines and;
FIGURE 2 is a simplified schematic diagram of an open line detector included in the system of Figure 1, and;
FIGURE 3 illustrates, at (a) to (c) several waveforms that can be present in the open line detector of Figure 1.

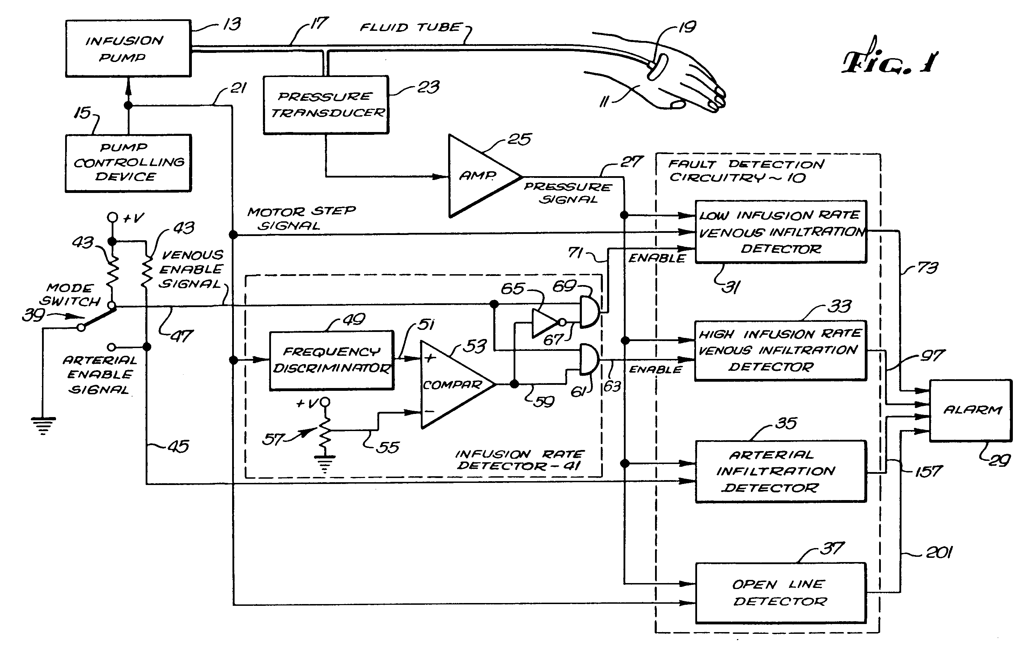

Referring now to the drawings, and particularly to Figure 1, there is shown fault detection circuitry 10 for use in a system for administering a parenteral fluid to the vascular system of a patient 11. The system includes a conventional infusion pump 13 and associated pump controlling device 15 for pumping the parenteral fluid through a fluid tube 17 and needle 19 to the patient. The pump is preferably of the peristaltic type, which pumps the fluid in a cyclic fashion. One such suitable pump and an associated controller for controlling its speed are described in a copending and commonly-assigned application for U.S. Patent Serial No. 06/281848 filed July 9, 1981, in the names of Stephen H. O'Leary et al and entitled "Method and Apparatus for Fluid Flow Control".

The pump controlling device 15 outputs a motor step signal for coupling on line 21 to the infusion pump 13. The signal is a sequence of pulses, each of which increments the pump by one step, to infuse a predetermined volume of parenteral fluid to the patient 11.

The parenteral administration system further includes a pressure transducer 23 and an associated amplifier 25 for monitoring the fluid pressure in the fluid tube 17 and producing a corresponding pressure signal for output on line 27.

The fault detection circuitry 10 evaluates the pressure signal on line 27 to detect certain characteristic patterns indicative of an improper fluid communication between the fluid tube 17 and the patient's vascular system. An alarm 29 is actuated if the circuitry detects such a condition. Such fault conditions include infiltrations of the fluid into body tissue other than the patient's vascular system, as well as a complete dislodging of the needle 19 from the patient 11 or a leak or air bubble in the fluid tube. In this way, a proper administration of the parenteral fluid can be ensured without the need for frequent monitoring or testing by hospital personnel.

More particularly, the fault detection circuitry 10 includes a low infusion rate venous infiltration detector 31 for detecting infiltrations when the infusion pump 13 is pumping parenteral fluid into the patient's venous system at a relatively low rate, and a high infusion rate venous infiltration detector 33 for detecting infiltrations when the pump is pumping fluid into the venous system at a relatively high rate. The fault detection circuitry further includes an arterial infiltration detector 35 for detecting infiltrations when the pump is pumping fluid into the patient's arterial system, and an open line detector 37 for detecting when there is a leak of some kind or an air bubble in the fluid connection between the pump and the patient 11.

The system further includes a mode switch 39 for indicating whether the system is intended to administer parenteral fluid to the patient's venous system or arterial system. The system also includes an infusion rate detector circuit 41, operable whenever the switch indicates that the system is pumping fluid into the patient's venous system to indicate whether the fluid is being pumped at a relatively high rate or a relatively low rate. The switch 39 and detector circuit 41 are used to enable operation of the appropriate venous infiltration detector circuit 31 or 33 or arterial infiltration detector circuit 35, depending on the system's operating mode.

More particularly, the mode switch 39 is a single-pole, double-throw switch having its middle terminal connected directly to ground and its two remaining terminals connected through separate resistors 43 to a positive voltage. The binary signals present on these two terminals are therefore opposite in phase to each other. One such signal is defined to be an arterial enable signal and the other is defined to be a venous enable signal.

The arterial enable signal is coupled on line 45 directly to the arterial infiltration detector circuit 35, and the venous enable signal is coupled on line 47 to the infusion rate detector 41. The infusion rate detector circuit relays the venous enable signal to either the low infusion rate venous infiltration detector 31 or the high infusion rate venous infiltration detector 33, depending on the infusion rate being effected by the infusion pump 13.

The infusion rate detector circuit 41 includes a frequency discriminator 49 for monitoring the motor step signal present on line 21 and producing an output signal having a voltage level generally proportional to the motor step signal's frequency. This output signal is coupled on line 51 to the positive input terminal of a comparator 53, which compares it with a selected reference level coupled to its negative input terminal. The reference level is supplied on line 55 from the wiper of a potentiometer 57, whose remaining two terminals are connected between ground and a positive supply voltage. If the discriminator output signal exceeds the threshold, indicating that the infusion pump 13 is pumping at a relatively high rate (e.g. above about 40 ml per hour), the comparator outputs a positive voltage level. On the other hand, if the discriminator output signal does not exceed the threshold, indicating that the pump is pumping at a relatively low rate, the comparator outputs a low voltage level signal.

The signal output by the comparator 53 is coupled on line 59 to a first AND gate 61 for ANDing with the venous enable signal supplied on line 47 from the mode switch 39. This produces a high infusion rate venous enable signal for coupling on line 63 to the high infusion rate venous infiltration detector 33. The detector 33 is thereby enabled to detect infiltrations whenever a venous infusion is selected by the mode switch and the infusion rate exceeds the prescribed threshold.

The signal output by the comparator 53 of the infusion rate detector 41 is also coupled on line 59 to a NOT gate 65, for inversion and coupling in turn on line 67 to a second AND gate 69, where it is ANDed with the same venous enable signal present on line 47. The resulting low infusion rate venous enable signal is coupled on line 71 to the low infusion rate venous infiltration detector 31. This detector 31 is thereby enabled whenever a venous infusion is selected by the mode switch 39 and the infusion rate does not exceed the prescribed threshold.

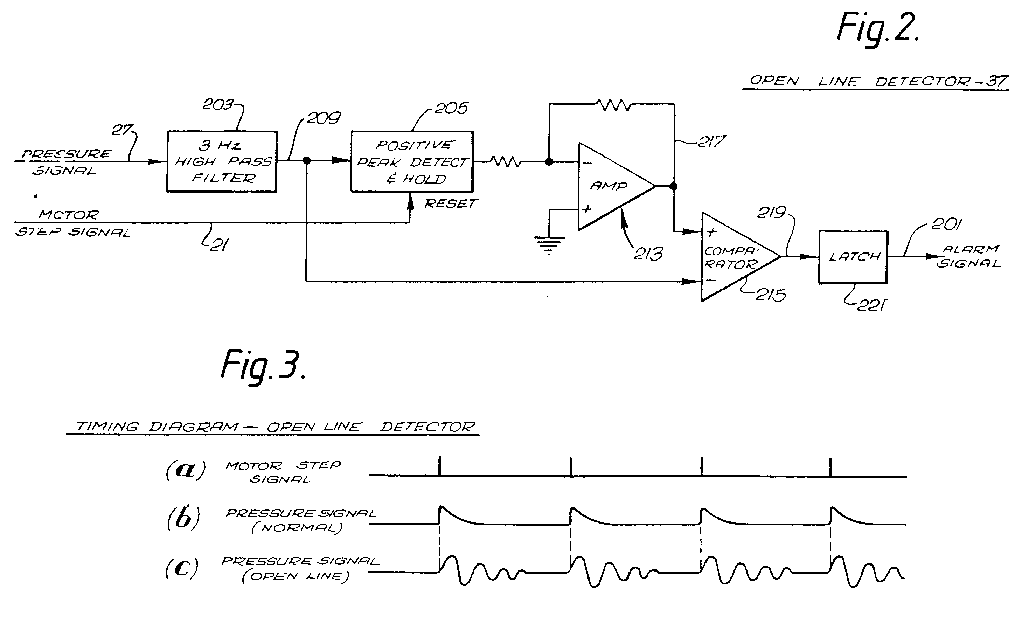

A simplified schematic diagram of the open line detector circuit 37 is depicted in Figure 2. This circuit monitors the pressure signal supplied on line 27 to detect a fluid leakage i.e. an open line or an air bubble in the fluid tube 17 between the infusion pump 13 and the patient 11. If the circuit detects such a condition, it outputs an alarm signal for coupling on line 201 to the alarm 29.

During normal operation, when the needle 19 is properly inserted into the patient's vein or artery, the pressure signal (Figure 3(b)) reflects an overdamped or critically damped condition. The signal includes a positive pressure pulse immediately following each pulse of the motor step signal (Figure 3(a)), with each pulse having an exponentially-decaying tail as the patient's vein or artery carries away the small volume of fluid being infused. Conversely, when an open line condition is present, the pressure signal (Figure 3(c)) reflects an underdamped condition. Each pulse of the motor step signal results in an initial increase in fluid pressure, followed immediately thereafter by a momentary decrease in pressure to a level less than the level before the pulse occurred.

Basically, the open line detector 37 detects the occurrence of an open line condition by detecting an underdamped characteristic in the pressure signal, i.e. an overshoot or ringing in the pressure signal following each pulse of the motor step signal. More particularly, the open line detector includes a 3Hz high-pass filter 203 and a positive peak detector and hold circuit 205. The high-pass filter filters the pressure signal supplied on line 27, to remove its d.c. level but pass the frequencies associated with the successive steps of the infusion pump 13. The filtered signal is coupled on line 209 to the peak detector and hold circuit, which outputs a level equal to the signal's positive peak. The peak detector circuit, which is reset by each pulse in the motor step signal supplied on line 21, can include for example, a series-connected diode followed by a capacitor to ground.

The signal output by the positive peak detector and hold circuit 205 is coupled on line 211 to an amplifier 213 for amplification by a prescribed fractional amount e.g. negative two-thirds. This amplified signal serves as a threshold to which the high-pass filtered pressure signal is compared in a comparator 215. If the filtered signal is ever more negative than the threshold, it is deduced that there is substantial ringing occurring in the pressure signal and that an open line condition is therefore present.

More particularly, the signal output by the amplifier 213 is coupled on line 217 to the comparator's positive input terminal and the high-pass filtered pressure signal is coupled on line 209 to the comparator's negative input terminal. The resulting comparator output signal is coupled on line 219 to a latch 221, which produces the alarm signal for coupling on line 201 to the alarm 29.

The alarm 29 is responsive to any of the four alarm signals coupled to it on lines 73, 97, 157 and 201 from the fault detection circuitry 10. It includes a four-input OR gate that OR's together the four signals and actuates a visual or audible indicator whenever any of the signals is high. It further includes a switch for use in selectively clearing the latches or flip-flops at the output stage of each detector in the fault detection circuitry.

It should be appreciated from the foregoing description that the apparatus described provides an improved apparatus and related method for detecting fault conditions such as an infiltration or an open line in a parenteral administration system. The apparatus is particularly adapted for use with a system of the type that includes a pulsing infusion pump for incrementally pumping a parenteral fluid through a fluid tube and needle to a patient's vascular system.

## Claims

1. A parenteral administration system having an infusion device (13) for infusing parenteral fluid in a pulsing fashion through a fluid tube (17) into the vascular system of a patient and having a fault detection apparatus which includes a pressure transducer means (23) for monitoring the pressure of the fluid in the fluid tube and producing a corresponding pressure signal and a processor for evaluating the pressure signal to detect the existence of a fault; characterised in that the processor includes filter means (203) for high-pass filtering the pressure signal to produce an a.c. pressure signal comprising a positive signal excursion occurring with each delivery pulse of the infusion device followed by a negative signal excursion, and comparison means (205, 213, 215) for comparing the magnitude of the negative signal excursion with a threshold comprising a predetermined proportion of the peak magnitude of the preceding positive signal excursion and alarm means (221, 201) for producing an alarm signal whenever the magnitude of the negative signal excursion exceeds said threshold.

2. A method of detecting a fault in a parenteral fluid infusion system, the infusion system having an infusion device (13) for infusing parenteral fluid in a pulsing fashion through a fluid tube (17) and further having pressure transducer means (23) for monitoring the pressure of the fluid in the fluid tube and for producing a corresponding pressure signal characterised by the steps of:
high-pass filtering the pressure signal to produce an a.c. pressure signal comprising a positive signal excursion occurring with each delivery pulse of the infusion device followed by a negative signal excursion;
determining a threshold by taking a predetermined proportion of the peak magnitude of said positive signal excursion;
comparing the magnitude of the negative signal excursion with the threshold; and
producing an alarm signal whenever the magnitude of the negative signal excursion exceeds said threshold.

## Patentansprüche

1. Ein parenterales Abgabesystem hat eine Infusionseinrichtung (13) zum Infundieren einer parenteralen Flüssigkeit in pulsierender Art und Weise durch eine Flüssigkeitsschlauch (17) in das vaskuläre System eines Patienten und mit einer Fehlererkennungsvorrichtung, die ein Druckwandmittel (23) zur Beobachtung des Drucks der Flüssigkeit in dem Flüssigkeitsschlauch aufweist und ein entsprechendes Drucksignal erzeugt, und mit einem Prozessor zum Bewerten des Drucksignals, um das Vorhandenseins eines Fehlzustandes zu erkennnen, dadurch gekennzeichnet, daß der Prozessor einen Filter (203) zum Hochpassfiltern des Drucksignals zur Erzeugung eines Wechselspannungsdrucksignals mit einer positiven Signalabweichung, die bei jedem Impuls der Infusionseinrichtung auftritt, dem eine negative Signalabweichung folgt, und Vergleichermittel (205, 213, 215) zum Vergleichen der Amplitude der negativen Signalabweichung mit einem Schwellenwert, der einem vorgegebenen Anteil der Peakamplitude der vorangehenden positiven Signalabweichung entspricht, und Alarmmittel (221, 201) zum Erzeugen eines Signals immer dann, wenn die Magnitude der negativen Signalabweichung den Schwellenwert übersteigt, aufweist.

2. Verfahren zum Erkennen eines Fehlers in einem parenteralen Infusionssystems, wobei das Infusionssystem eine Einrichtung (13) zum Infundieren einer parenteralen Flüssigkeit in pulsierender Weise durch einen Flüssigkeitsschlauch (17) und weiter Druckwandelmittel (23) zum Beobachten des Drucks in der Flüssigkeit in dem Flüssigkeitsschlauch und zum Erzeugen eines entsprechenden Drucksignals aufweist, gekennzeichnet durch die folgenden Schritte:
- Hochpassfiltern des Drucksignals zum Erzeugen eines Wechselspannungsdrucksignals, das eine positive Signalabweichung aufweist, die bei jedem Vorliegen eines Impulses der Infusionseinrichtung auftritt, gefolgt von einer negativen Signalabweichung beinhaltet,
- Bestimmen eines Schwellenwertes durch Nehmen eines vorgegebenen Anteils der Peakamplitude der positiven Signalabweichung;
- Vergleichen der Magnitude der negativen Signalabweichung mit dem Schwellenwert; und
- Erzeugen eines Alarmsignals immer dann, wenn die Amplitude der negativen Signalabweichung den Schwellenwert übersteigt.

## Revendications

1. Système d'administration parentérale comportant un dispositif de perfusion (13) destiné à perfuser un fluide parentéral d'une manière pulsée par un tube de fluide (17) dans le système vasculaire d'un patient et comportant un appareil de détection de défauts qui comprend un transducteur de pression 23 destiné à surveiller la pression du fluide dans le tube de fluide et à produire un signal de pression correspondant et un processeur destiné à évaluer le signal de pression pour détecter l'existence d'un défaut; caractérisé en ce que le processeur comprend des moyens de filtrage (203) pour le filtrage passe-haut du signal de pression pour produire un signal de pression courant alternatif comprenant une excursion de signal positif se produisant à chaque impulsion d'alimentation du dispositif de perfusion suivie d'une excursion de signal négatif et des moyens de comparaison (205, 213, 215) destinés à comparer l'ampleur de l'excursion du signal négatif avec un seuil comprenant une proportion prédéterminée de l'ampleur de pic de l'excursion de signal positif précédent et des moyens d'alarme (221, 201) destinés à produire un signal d'alarme chaque fois que l'ampleur de l'excursion du signal négatif dépasse ce seuil.

2. Procédé de détection d'un défaut dans un système de perfusion parentérale, le système de perfusion comportant un dispositif de perfusion (13) destiné à perfuser un fluide parentéral d'une manière pulsée par un tube de fluide (17) et comportant de plus un transducteur de pression (23) destiné à surveiller la pression du fluide dans le tube de fluide et à produire un signal de pression correspondant caractérisé par les étapes de :
- filtrage passe-haut du signal de pression pour produire un signal de pression courant alternatif comprenant une excursion de signal positif se produisant à chaque impulsion d'alimentation du dispositif de perfusion suivie d'une excursion de signal négatif;
- détermination d'un seuil en prenant une proportion prédéterminée de l'ampleur de pic de l'excursion de signal positif;
- comparaison de l'ampleur de l'excursion de signal négatif avec le seuil; et
- production d'un signal d'alarme chaque fois que l'ampleur de l'excursion du signal négatif dépasse le seuil.
